# DEMANDE DE BREVET EUROPEEN

(11) **EP 3 143 981 A1**
(43) Date de publication de la demande: **22.03.2017**
(21) Numéro de dépôt: 15185565.7
(22) Date de dépôt: 16.09.2015
(51) Int. Cl.: A61K 8/31, A61Q 19/00, A61K 8/06

(54) **COMPOSITION ÉMOLLIENTE BIOSOURCÉE**

(71) Demandeur: TOTAL MARKETING SERVICES, 92800 Puteaux (FR)
(72) Inventeur: SWOBODA, Benjamin., 78630 Orgeval (FR)
(74) Mandataire: August & Debouzy avocats

(57) **Abrégé**

Composition émolliente comprenant au moins une huile hydrocarbonée qui comprend une teneur en isoparaffines allant de 90 à 100% en poids, une teneur en paraffines normales allant de 0 à 10% en poids et une teneur en carbone d'origine biologique supérieure ou égale à 90 % par rapport au poids total de l'huile hydrocarbonée.

## Description

### DOMAINE DE L'INVENTION

L'invention concerne une composition émolliente comprenant au moins une huile hydrocarbonée biodégradable, d'origine biologique et majoritairement isoparaffinique.

L'invention concerne également une composition cosmétique, dermatologique ou pharmaceutique comprenant ladite composition émolliente et son utilisation.

La présente invention concerne aussi l'utilisation de ladite composition émolliente pour la formulation de produits cosmétiques, de produits dermatologiques ou de produits pharmaceutiques.

### CONTEXTE TECHNIQUE DE L'INVENTION

Les marchés de la cosmétique, de la dermatologie ou encore de la pharmacie sont de plus en plus demandeurs d'ingrédient d'origine biologique pour la formulation de leur produis. Alors que les actifs, les émulsifiants et les huiles végétales biosourcés ont été fortement développés ces dernières années et sont maintenant largement disponibles sur le marché, les émollients biosourcés, surtout les émollients hydrocarbonés d'origine 100% biologique, restent encore rares.

Les émollients actuellement utilisés en cosmétique sont soit des isoparaffines issue de la pétrochimie (principalement l'isododécane et l'isohexadécane), des huiles blanches, des huiles silicones ou des huiles à base d'esters (synthétiques ou naturelles). Les isoparaffines, les huiles blanches et les huiles silicones sont largement diffusées car elles sont très stables et sans odeur mais ne sont pas issues de ressource renouvelable. Bien que les silicones volatiles telles que la cyclomethicone aient été longtemps considérées comme des émollients et solvants inoffensifs pour la peau (International Journal of Toxicology, Vol. 10, n°1, pp. 9-19, 1991), des craintes se sont exprimées ces dernières années concernant leurs potentiels effets délétères sur l'environnement, voire sur la santé humaine (en particulier en ce qui concerne l'octaméthylcyclotétrasiloxane). Les huiles à base d'ester sont dans certains cas issues de ressources renouvelables mais peu stables et avec une forte odeur du fait de leur structure chimique insaturée.

A ce jour, lorsque les industriels souhaitent formuler des produits comprenant des émollients comportant une connotation « bio », ils s'orientent généralement vers des émollients synthétiques à base d'esters, vers des huiles végétales ou vers des substances dites pures. La demande de brevet US2007260102 décrit par exemple un procédé d'hydrotraitement d'une huile végétale et d'une huile minérale pour l'obtention d'un solvant comprenant plus de 98% de paraffines normales utilisable dans des compositions cosmétiques. De la même manière la demande de brevet WO2011146837 décrit un procédé de préparation de composés purs utilisables en cosmétiques, le squalane et l'isosqualane, à partir de l'hydrogénation du β-farnésène. La demande de brevet FR294428 décrit une composition huileuse volatile utilisée en cosmétique, pour le remplacement des huiles de silicones et des isoparaffines, composée de paraffines linéaires et obtenue par déshydratation d'alcools gras d'origine végétale. Ces types d'émollients ont des désavantages qui limitent leur application et leur développement. Ces inconvénients sont par exemple l'instabilité avec de mauvaises propriétés en vieillissement, une odeur prononcée peu acceptable et/ou encore un prix élevé.

Il subsiste donc le besoin de disposer d'une composition émolliente biosourcée présentant des caractéristiques physico-chimiques et sensorielles notamment en termes de stabilité, de volatilité, d'étalement sur la peau, d'odeur, de toucher doux et de brillance, la rendant hautement compatible avec une utilisation en formulation.

La demanderesse a trouvé de manière surprenante que ce besoin peut être satisfait par une nouvelle composition émolliente d'origine biologique comprenant majoritairement des isoparaffines et permettant de réaliser des compositions stables aux propriétés physico-chimiques et sensorielles améliorées.

La présente invention a pour objectif de fournir une composition émolliente isoparaffinique issue de matière première d'origine biologique.

La présente invention a pour objectif de fournir une composition émolliente aux propriétés physico-chimiques et sensorielles améliorées.

La présente invention a également pour objectif de proposer une composition émolliente stable aux propriétés adaptées à son utilisation.

### RÉSUMÉ DE L'INVENTION

Ces objectifs sont atteints grâce à une nouvelle composition émolliente.

L'invention concerne une composition émolliente comprenant au moins une huile hydrocarbonée qui comprend une teneur en poids d'isoparaffines allant de 90 à 100%, une teneur en poids de paraffines normales allant de 0 à 10% et une teneur en carbone d'origine biologique supérieure ou égale à 90 % par rapport au poids total de l'huile hydrocarbonée.

De préférence, l'huile hydrocarbonée de la composition émolliente de l'invention comprend une teneur en carbone d'origine biologique supérieure ou égale à 95%, de préférence supérieure ou égale à 98% et préférentiellement de 100% par rapport au poids total de l'huile hydrocarbonée.

De préférence, l'huile hydrocarbonée de la composition émolliente de l'invention comprend une teneur en poids d'isoparaffines allant de 90 à 100%, préférentiellement de 95 à 100% et plus préférentiellement de 98% à 100% par rapport au poids total de l'huile hydrocarbonée.

De préférence, l'huile hydrocarbonée de la composition émolliente de l'invention est choisie parmi les isoparaffines non-cycliques comprenant 14 à 18 atomes de carbone.

De préférence, l'huile hydrocarbonée de la composition émolliente de l'invention comprend une teneur en poids de paraffines normales inférieure ou égale à 10%, préférentiellement inférieure ou égale à 5% et plus préférentiellement inférieure ou égale à 2% par rapport au poids total de l'huile hydrocarbonée.

De préférence, l'huile hydrocarbonée de la composition émolliente de l'invention comprend une teneur en poids de composés naphténiques inférieure ou égale à 1%, préférentiellement inférieure ou égale à 0,5% et plus préférentiellement inférieure ou égale à 100 ppm par rapport au poids total de l'huile hydrocarbonée.

De préférence, l'huile hydrocarbonée de la composition émolliente de l'invention comprend une teneur en poids de composés aromatiques inférieure ou égale à 500 ppm, préférentiellement inférieure ou égale à 300 ppm et plus préférentiellement inférieure ou égale à 100 ppm par rapport au poids total de l'huile hydrocarbonée.

De préférence, l'huile hydrocarbonée de la composition émolliente de l'invention a une température d'ébullition allant de 230 à 340°C, préférentiellement de 235 à 330°C et plus préférentiellement de 240 à 325°C selon la norme ASTM D86.

De préférence, l'huile hydrocarbonée de la composition émolliente de l'invention est obtenue par un procédé d'hydrogénation catalytique à une température de 80 à 180°c et à une pression de 50 à 160 bars d'une charge d'origine biologique désoxygénée et/ou isomérisée.

Avantageusement, l'huile hydrocarbonée de la composition émolliente de l'invention a une biodégradabilité à 28 jours d'au moins 60%, préférentiellement d'au moins 70%, plus préférentiellement d'au moins 75% et encore plus préférentiellement d'au moins 80% mesurée selon la norme OECD 306.

De préférence, l'huile hydrocarbonée de la composition émolliente de l'invention a un point éclair supérieur ou égal à 110°C selon la norme ASTM D93.

De préférence, l'huile hydrocarbonée de la composition émolliente de l'invention a une pression de vapeur à 20°C inférieure ou égale à 0,01kPa.

L'invention a également pour objet une composition cosmétique, dermatologique ou pharmaceutique comprenant au moins une composition émolliente de l'invention, de préférence en une quantité allant de 0,5 à 80%, préférentiellement de 1 à 50% et avantageusement de 5 à 30% en poids par rapport au poids total de la composition.

Selon un mode de réalisation la composition cosmétique, dermatologique ou pharmaceutique de l'invention comprenant au moins un corps gras choisi parmi : les huiles végétales, les huiles hydrocarbonées, les beurres végétaux, les éthers et alcools gras, les esters huileux et alcanes et les huiles silicones et/ou au moins un additif.

Un autre objet de l'invention est l'utilisation cosmétique de la composition émolliente de l'invention ou de la composition cosmétique, dermatologique ou pharmaceutique de l'invention pour une application topique, notamment comme produit de soin pour la peau, comme produit de maquillage, comme produit capillaire, comme produit démaquillant, comme produit parfumé, comme produit solaire.

Un objet de l'invention est encore une utilisation pharmaceutique de la composition émolliente de l'invention ou de la composition cosmétique, dermatologique ou pharmaceutique de l'invention.

Un autre objet de l'invention est aussi l'utilisation de la composition émolliente de l'invention ou de la composition cosmétique, dermatologique ou pharmaceutique de l'invention pour la formulation de produits cosmétiques, de produits dermatologiques ou de produits pharmaceutiques.

L'invention a également pour objet un procédé de traitement cosmétique de la peau comprenant au moins une étape d'application, de préférence par étalement, de la composition émolliente de l'invention ou de la composition cosmétique, dermatologique ou pharmaceutique de l'invention.

### DESCRIPTION DETAILLÉE DE L'INVENTION

L'invention concerne une composition émolliente comprenant au moins une huile hydrocarbonée qui comprend une teneur en poids d'isoparaffines allant de 90 à 100%, une teneur en poids de paraffines normales allant de 0 à 10% et une teneur en carbone d'origine biologique supérieure à 95% par rapport au poids total de l'huile hydrocarbonée.

La composition émolliente selon l'invention permet de disposer d'une composition non-irritante, non-odorante et biodégradable.

La composition émolliente selon l'invention permet en particulier d'obtenir des compositions cosmétiques, dermatologiques ou pharmaceutiques stables.

La composition émolliente selon l'invention présente par ailleurs un caractère émollient et un étalement facile sur la peau de sorte que les compositions cosmétiques, dermatologiques ou pharmaceutiques formulées laissent subsister sur la peau, après pénétration dans ses couches externes, un film ayant un toucher doux et non gras.

A titre préliminaire on notera que, dans la description et les revendications suivantes, l'expression « compris entre » doit s'entendre comme incluant les bornes citées.

### Composition émolliente :

La composition émolliente selon l'invention comprend de préférence une teneur d'huile hydrocarbonée allant de 50 à 100% en poids, préférentiellement de 80 à 100% en poids, plus préférentiellement de 90 à 100% en poids et avantageusement égale à 100% en poids par rapport au poids total de la composition.

La présence d'une quantité significative d'huile hydrocarbonée selon l'invention contribue aux qualités cosmétique, pharmaceutique et/ou dermatologique de la composition émolliente : toucher agréable, soin, brillance et protection de la peau.

L'huile hydrocarbonée de la composition émolliente selon l'invention comprend de préférence une teneur en poids de composés isoparaffiniques supérieure ou égale à 90%, préférentiellement supérieure ou égale à 95 % et avantageusement supérieure ou égale à 98% par rapport au poids total de l'huile hydrocarbonée.

L'huile hydrocarbonée de la composition émolliente selon l'invention comprend de préférence une teneur en poids de paraffines normales inférieure ou égale à 10%, préférentiellement inférieure ou égale à 5 % et avantageusement inférieure ou égale à 2%.

L'huile hydrocarbonée de la composition émolliente selon l'invention comprend avantageusement une majorité de d'isoparaffines et une minorité de paraffines normales. Ces isoparaffines sont avantageusement des isoparaffines non-cycliques. De préférence l'huile hydrocarbonée de la composition émolliente a un ratio d'isoparaffines sur paraffines normales d'au moins 12 :1, préférentiellement de 15 :1 et plus préférentiellement de 20 :1. De manière encore plus avantageuse l'huile hydrocarbonée de la composition émolliente selon l'invention ne contient pas de paraffines normales.

Selon un mode de réalisation, l'huile hydrocarbonée selon l'invention comprend de préférence une teneur en poids d'isoparaffines allant de 90 à 100% et une teneur en paraffines normales allant de 0 à 10%, préférentiellement de 95 à 100% d'isoparaffines et de 0 à 5% de paraffines normales et plus préférentiellement de 98% à 100% d'isoparaffines et de 0 à 2 % de paraffines normales.

L'huile hydrocarbonée de la composition émolliente selon l'invention comprend de préférence une teneur en poids de composés naphténiques inférieure ou égale à 1%, préférentiellement inférieure ou égale à 0.5% et plus préférentiellement inférieure ou égale à 100ppm.

Selon un autre mode de réalisation préféré, l'huile hydrocarbonée de la composition émolliente selon l'invention comprend une teneur en poids d'isoparaffines allant de 90 à 100%, une teneur en poids de paraffines normales allant de 0 à 10% et une teneur en poids de naphtènes inférieure ou égale à 1%. Préférentiellement l'huile hydrocarbonée comprend une teneur en poids allant de 95 à 100% d'isoparaffines, de 0 à 5% de paraffines normales et une teneur en poids de naphtènes inférieure ou égale à 0.5%. Plus préférentiellement elle comprend une teneur en poids allant de 98% à 100% d'isoparaffines, de 0 à 2 % de paraffines normales et une teneur en poids de naphtènes inférieure ou égale à 100ppm.

L'huile hydrocarbonée mise en oeuvre dans la composition émolliente selon l'invention est avantageusement exempte de composés aromatiques. Par exempte, on entend une teneur en poids de composés aromatiques inférieure ou égale à 500 ppm, de préférence inférieure ou égale à 300 ppm, préférentiellement inférieure ou égale à 100 ppm, plus préférentiellement inférieure ou égale à 50 ppm et avantageusement inférieure ou égale à 20 ppm mesurée par spectrométrie UV.

Selon un autre mode de réalisation préféré, l'huile hydrocarbonée de la composition émolliente selon l'invention comprend une teneur en poids d'isoparaffines allant de 90 à 100%, une teneur en poids de paraffines normales allant de 0 à 10%, une teneur en poids de naphtènes inférieure ou égale à 1% et une teneur en poids de composés aromatiques inférieure ou égale à 500 ppm. Préférentiellement l'huile hydrocarbonée comprend une teneur en poids allant de 95 à 100% d'isoparaffines, de 0 à 5% de paraffines normales, une teneur en poids de naphtènes inférieure ou égale à 0.5% et une teneur en poids de composés aromatiques inférieure ou égale à 300ppm. Plus préférentiellement elle comprend une teneur en poids allant de 98% à 100% d'isoparaffines, de 0 à 2 % de paraffines normales, une teneur en poids de naphtènes inférieure ou égale à 100ppm et une teneur en poids de composés aromatiques inférieure ou égale à 100 ppm.

L'huile hydrocarbonée mise en oeuvre dans la composition émolliente selon l'invention a également de préférence une teneur en poids de composés soufrés extrêmement basse, typiquement inférieure ou égale à 5ppm, préférentiellement inférieure ou égale à 3 ppm et plus préférentiellement inférieure ou égale à 0.5 ppm à un niveau trop bas pour être détectée grâce à des analyseurs de basse-teneur en soufre conventionnels.

L'huile hydrocarbonée mise en oeuvre dans la composition émolliente selon l'invention a également de préférence un point éclair supérieur ou égal à 110°C, préférentiellement supérieur ou égal à 120°C et plus préférentiellement supérieur ou égal à 140°C selon la norme ASTM D93. Un point éclair élevé, typiquement supérieure à 110°C permettant entre autre de palier d'une part les problèmes de sécurité lors du stockage et du transport en évitant une inflammabilité trop sensible de l'huile hydrocarbonée.

L'huile hydrocarbonée a aussi de préférence une pression de vapeur à 20°C inférieure ou égale à 0.01kPa.

Selon un mode de réalisation, l'huile hydrocarbonée mise en oeuvre dans la composition émolliente selon l'invention a également de préférence un point éclair supérieur ou égal à 110°C selon la norme ASTM D93 et une pression de vapeur à 20°C inférieure ou égale à 0.01kPa. Préférentiellement l'huile hydrocarbonée a un point éclair supérieur ou égal supérieur ou égal à 120°C et une pression de vapeur à 20°C inférieure ou égale à 0.01kPa.Et plus préférentiellement, elle a un point éclair supérieur ou égal à 140°C et une pression de vapeur à 20°C

Il a aussi été découvert que lorsque les mélanges paraffiniques ont une température d'ébullition en dessous de 185°C, leur point éclair devient trop bas, les rendant trop dangereux car trop sensibles à l'inflammation. Il a aussi été découvert qu'au contraire lorsque le point d'ébullition de tels mélanges excèdent 215°C, la volatile de l'huile, définit par la pression de vapeur, devient trop basse rendant la composition émolliente désagréable à l'utilisation car des résidus apparaissent après application. L'invention offre donc une solution à la problématique précédente.

L'huile hydrocarbonée mise en oeuvre dans la composition émolliente selon l'invention présente des températures d'ébullition, un point éclair et une pression de vapeur permettant de pallier les problèmes d'inflammabilité, d'odeur et de volatilité.

L'huile hydrocarbonée de la composition émolliente selon l'invention a en outre de préférence une viscosité cinématique à 40°C inférieure ou égale à 5 cSt, préférentiellement inférieure ou égale à 4 cSt et plus préférentiellement inférieure ou égale à 4 cSt selon la norme ASTM D445.

### Procédé d'obtention :

De telles compositions d'huiles hydrocarbonées peuvent être obtenues de la façon suivante. L'huile hydrocarbonée selon l'invention est une coupe hydrocarbonée qui est issue de la conversion de la biomasse.

Par issue de la conversion de la biomasse, on entend une coupe hydrocarbonée produite à partir de matières premières d'origine biologique.

De préférence, La coupe hydrocarbonée d'origine biologique est obtenue par un procédé comprenant des étapes d'hydrodésoxygénation (HDO) et d'isomérisation (ISO). L'étape d'hydrodésoxygénation (HDO) conduit à la décomposition des structures des esters biologiques ou des constituants triglycérides, à l'élimination des composés oxygénés, phosphorés et soufrés et à l'hydrogénation des liaisons oléfiniques. Le produit issu de la réaction d'hydrodésoxygénation est ensuite isomérisé. Une étape de fractionnement peut de préférence suivre les étapes d'hydrodésoxygénation et d'isomérisation. De manière avantageuse, les fractions d'intérêt sont ensuite soumises à des étapes d'hydrotraitement puis de distillation afin obtenir les spécifications de l'huile hydrocarbonée souhaitée selon l'invention.

Ce procédé HDO/ISO est mise en oeuvre sur une charge biologique brute, encore appelée biomasse ou matière première d'origine biologique, sélectionnée dans le groupe consistant en des huiles végétales, des graisses animales, des huiles de poisson et leur mélange. Les matières premières d'origine biologique appropriées sont par exemple l'huile de colza, l'huile de canola, le talloil, l'huile de tournesol, l'huile de soja, l'huile de chanvre, l'huile d'olive, l'huile de lin, l'huile de moutarde, l'huile de palme, l'huile d'arachide, l'huile de ricin, l'huile de noix de coco, les graisses animales telles que le suif, les graisses alimentaires recyclées, les matières premières issues du génie génétique, et les matières premières biologiques produites à partir de microorganismes tels que les algues et les bactéries. Des produits de condensation, esters ou autres dérivés obtenus à partir de matériaux biologiques bruts peuvent également servir de matières premières.

De préférence, la matière première d'origine biologique est un ester ou un dérivé triglycéride. Ce matériaux est soumis tout d'abord à une étape d'hydrodésoxygénation (HDO) pour décomposer la structure des esters ou triglycérides constitutifs et éliminer les composés oxygénés, phosphorés et soufrés de manière concomitante à l'hydrogénation des liaisons oléfiniques. Cette étape d'hydrodésoxygénation (HDO) de la matière première d'origine biologique est suivie par une isomérisation du produit ainsi obtenu conduisant à la ramification de la chaîne hydrocarbonée et à une amélioration des propriétés de la paraffine à basses températures.

Durant l'étape HDO, l'hydrogène et la matière première d'origine biologique sont passés sur un lit catalytique d'hydrodésoxygénation de manière simultanée ou à contre courant. Durant l'étape HDO, la pression et la température sont comprises entre 20 et 150 bars et entre 200 et 500°C respectivement. Des catalyseurs classiques et connus d'hydrodésoxygénation sont utilisés durant cette étape. Éventuellement, la matière première d'origine biologique peut être soumise à une pré-hydrogénation sous conditions douces pour éviter les réactions secondaires des doubles liaisons avant l'étape HDO. Après l'étape d'hydrodésoxygénation, le produit issu de la réaction est soumis à une étape isomérisation (ISO) où l'hydrogène et le produit, et éventuellement un mélange de n-paraffines, sont passés sur des lits catalytiques d'isomérisation de manière simultanée ou à contre-courant. Lors de l'étape ISO, la pression et la température sont comprises entre 20 et 150 bars et entre 200 et 500°C respectivement. Des catalyseurs classiques et connus d'isomérisation sont utilisés durant cette étape.

Des procédés secondaires additionnels peuvent également être mise en oeuvre (comme des mélanges intermédiaires, des piégeages ou autres procédés de la sorte).

Le produit issu des étapes HDO/ISO peut éventuellement être fractionné afin d'obtenir les coupes d'intérêt.

Divers procédés HDO/ISO sont décrits dans la littérature. La demande WO2014/033762 décrit un procédé comprenant une étape de pré-hydrogénation, une étape d'hydrodésoxygénation (HDO) et une étape d'isomérisation opérées à contre-courant. La demande de brevet EP1728844 décrit un procédé de production de composés hydrocarbonés à partir d'un mélange de composés d'origine végétale et animale. Ce procédé comprend une étape de prétraitement du mélange permettant d'enlever les contaminants, comme par exemple les sels de métaux alcalins, suivie d'une étape d'hydrodésoxygénation (HDO) et d'une étape d'isomérisation. La demande de brevet EP2084245 décrit un procédé de production d'un mélange hydrocarboné qui peut être utilisé comme gazole ou dans une composition de gazole par hydrodésoxygénation d'un mélange d'origine biologique contenant des esters d'acides gras éventuellement en mélange avec des acides gras libres, par exemple des huiles végétales comme l'huile de tournesol, l'huile de colza, l'huile de canola, l'huile de palme ou l'huile de pin, suivi d'une hydroisomérisation sur des catalyseurs spécifiques. La demande de brevet EP2368967 décrit un tel procédé et le produit obtenu par ce procédé.

Avantageusement, la matière première d'origine biologique contient moins de 15 ppm de soufre, de préférence moins de 8ppm, préférentiellement moins de 5ppm et plus préférentiellement moins de 1ppm selon la norme EN ISO 20846. Idéalement la charge ne comprend pas de soufre en tant que matière première d'origine biosourcée.

Avant l'étape d'hydrotraitement, une étape de préfractionnement peut avoir lieu. Une coupe plus étroite en entrée d'unité d'hydrogénation permet d'obtenir une coupe étroite en sortie d'unité. En effet, les points d'ébullition de coupes préfractionnées sont compris entre 220 et 330 °C tandis que les coupes qui n'ont pas été préfractionnées ont typiquement des points d'ébullition comprise entre 150 et 360°C.

La charge désoxygénée et isomérisée issue du procédé HDO/ISO est ensuite hydrogénée.

L'hydrogène utilisé dans l'unité d'hydrogénation est typiquement de l'hydrogène hautement purifié. On entend par hautement purifié, de l'hydrogène d'une pureté par exemple supérieure à 99%, même si d'autres grades peuvent également être utilisés.

L'étape d'hydrogénation est effectuée grâce à des catalyseurs. Les catalyseurs d'hydrogénation types peuvent être soit massiques soit supportés et peuvent comprendre les métaux suivants : nickel, platine, palladium, rhénium, rhodium, tungstate de nickel, nickel-molybdène, molybdène, cobalt-molybdène. Les supports peuvent être de la silice, de l'alumine, de la silice-alumine ou des zéolithes.

Un catalyseur préféré est un catalyseur à base de nickel sur support d'alumine dont l'aire de surface spécifique varie entre 100 et 200 m2/g de catalyseur ou un catalyseur massique à base de nickel. Les conditions d'hydrogénation sont typiquement les suivantes :
- Pression : 50 à 160 bars, de préférence 80 à 150 bars et plus préférentiellement 90 à 120 bars ;
- Température : 80 à 180 °C, de préférence 120 à 160 °C et plus préférentiellement 150 à 160 °C ;
- Vitesse volumique horaire (VVH): 0.2 à 5 hr⁻¹, de préférence 0.4 à 3 hr⁻¹ et plus préférentiellement 0.5 à 0.8 hr⁻¹ ;
- Taux de traitement par l'hydrogène : adapté aux conditions mentionnées ci-dessus et pouvant aller jusqu'à 200 Nm³/tonnes de charge à traiter.

La température dans les réacteurs est typiquement comprise entre 150 et 160°C avec une pression d'environ 100 bars tandis que la vitesse volumique horaire est d'environ 0.6 hr⁻¹ avec un taux de traitement adapté en fonction de la qualité de la charge à traiter et des paramètres du premier réacteur d'hydrogénation.

L'hydrogénation peut avoir lieu dans un ou plusieurs réacteurs en série. Les réacteurs peuvent comprendre un ou plusieurs lits catalytiques. Les lits catalytiques sont généralement des lits catalytiques fixes.

Le procédé d'hydrogénation comprend de préférence deux ou trois réacteurs, de préférence trois réacteurs et est plus préférentiellement réalisé dans trois réacteurs en série.

Le premier réacteur permet le piégeage des composés soufrés et l'hydrogénation d'essentiellement tous les composés insaturés et jusqu'à environ 90 % des composés aromatiques. Le produit issu du premier réacteur ne contient substantiellement aucun composé soufré. Au second stade c'est-à-dire dans le second réacteur, l'hydrogénation des aromatiques se poursuit et jusqu'à 99 % des aromatiques sont de ce fait hydrogénés.

Le troisième stade dans le troisième réacteur est un stade de finition permettant d'obtenir des teneurs en aromatiques inférieures ou égales à 500 ppm, de préférence inférieures ou égales à 300 ppm, préférentiellement inférieures ou égales à 100 ppm, plus préférentiellement inférieures ou égales à 50 ppm, et idéalement inférieures ou égales à 20ppm même dans le cas de produits à haut point d'ébullition par exemple supérieur à 300°C.

Il est possible d'utiliser un réacteur qui comporte deux ou trois lits catalytiques ou plus. Les catalyseurs peuvent être présents à des quantités variables ou essentiellement égales dans chaque réacteur ; pour trois réacteurs, les quantités en fonction du poids peuvent par exemple être de 0,05-0,5/0,10-0,70/0,25-0,85, de préférence 0,07-0,25/0,15-0,35/0,4-0,78 et plus préférentiellement de 0,10-0,20/0,20-0,32/0,48-0,70.

Il est également possible d'utiliser un ou deux réacteurs d'hydrogénation au lieu de trois.

Il est également possible que le premier réacteur soit composé de réacteurs jumeaux mis en oeuvre de manière alternative. Ce mode d'opérabilité permet notamment un chargement et un déchargement facilité des catalyseurs : lorsque le premier réacteur comprend le catalyseur saturé en premier (substantiellement tout le soufre est piégé sur et/ou dans le catalyseur) il doit être changé souvent.

Un seul réacteur peut également être utilisé dans lequel deux, trois lits catalytiques ou plus sont installés.

Il peut être nécessaire d'insérer des boîtes de quench (au sens anglais « d'étouffement de la réaction ») dans le système de recycle ou entre les réacteurs pour refroidir les effluents d'un réacteur à un autre ou d'un lit catalytique à un autre afin de contrôler les températures et l'équilibre hydrothermique de chaque réaction. Selon un mode de réalisation préféré, il n'y a pas d'intermédiaires de refroidissement ou d'étouffement.

Selon un mode de réalisation, le produit issu du procédé et/ou les gaz séparés sont au moins en partie recyclé(s) dans le système d'alimentation des réacteurs d'hydrogénation. Cette dilution contribue à maintenir l'exothermicité de la réaction dans des limites contrôlées, en particulier au premier stade. Le recyclage permet en outre un échange de chaleur avant la réaction et aussi un meilleur contrôle de la température.

L'effluent de l'unité d'hydrogénation contient principalement le produit hydrogéné et de l'hydrogène. Des séparateurs flash sont utilisés pour séparer les effluents en phase gazeuse, principalement l'hydrogène résiduel, et en phase liquide, principalement les coupes hydrocarbonées hydrogénés. Le procédé peut être effectué en utilisant trois séparateurs flash, un à pression élevée, un à pression intermédiaire et un à basse pression très proche de la pression atmosphérique.

L'hydrogène gazeux qui est recueilli en haut des séparateurs flash peut être recyclé dans le système d'alimentation de l'unité d'hydrogénation ou à différents niveaux dans les unités d'hydrogénation entre les réacteurs.

Selon un mode de réalisation, le produit final est séparé à pression atmosphérique. Il alimente ensuite directement une unité de fractionnement sous vide. De préférence, le fractionnement se fera à une pression comprise entre 10 et 50 mbars et plus préférentiellement à environ 30 mbars.

Le fractionnement peut être effectué de façon à ce qu'il soit possible de retirer simultanément divers fluides hydrocarbonés de la colonne de fractionnement et à ce que leur température d'ébullition puisse être prédéterminée.

En adaptant la charge au travers de ses points d'ébullition initiaux et finaux, les réacteurs d'hydrogénation, les séparateurs et l'unité de fractionnement peuvent donc être directement connectés sans qu'il soit nécessaire d'utiliser des cuves intermédiaires. Cette intégration de l'hydrogénation et du fractionnement permet une intégration thermique optimisée associée à une réduction du nombre d'appareils et à une économie d'énergie.

L'huile hydrocarbonée mise en oeuvre dans la composition émolliente de l'invention est avantageusement une coupe hydrocarbonée ayant un intervalle de distillation ID (en °C) allant de 230°C à 340°C, de préférence de 235°C à 330°C et plus préférentiellement de 240°C à 325°C mesuré selon la norme ASTM D86. De préférence, la différence entre le point d'ébullition initial et le point d'ébullition final est inférieure ou égale à 80°C, préférentiellement inférieure ou égale à 70°C, plus préférentiellement inférieure ou égale à 60°C et avantageusement comprise entre 40 et 50°C. L'huile hydrocarbonée peut comprendre une ou plusieurs fractions d'intervalles de distillation compris dans les intervalles décrits ci-dessus.

Avantageusement, l'huile hydrocarbonée mise en oeuvre dans la composition émolliente de l'invention est totalement saturée. De préférence, les composants de l'huile hydrocarbonée sont choisis parmi les isoparaffines comprenant 13 à 27 atomes de carbone, préférentiellement 13 à 18 atomes de carbone et plus préférentiellement 14 à 18 atomes de carbones.

La composition émolliente selon l'invention comprend avantageusement une teneur en isohexadécane inférieure ou égale à 50%.

L'huile hydrocarbonée de la composition émolliente selon l'invention est idéalement issue du traitement de matières premières d'origine biologique. Le terme de «bio-carbone" indique que le carbone est d'origine naturelle et vient d'un biomatériau, comme indiqué ci-après. La teneur en bio-carbone et la teneur en biomatériau sont des expressions indiquant la même valeur. Un matériau d'origine renouvelable ou biomatériau est un matériau organique dans lequel le carbone est issu du CO₂ fixé récemment (sur une échelle humaine) par photosynthèse à partir de l'atmosphère. Un biomatériau (Carbone 100% d'origine naturel) présente un rapport isotopique ¹⁴C /¹²C supérieure à 10⁻¹², typiquement environ 1,2 x 10⁻¹², tandis qu'un matériau fossile a un rapport nul. En effet, le ¹⁴C isotopique est formé dans et l'atmosphère est alors intégré par photosynthèse, selon une échelle de temps de quelques des dizaines d'années au maximum. La demi-vie du ¹⁴C est 5730 années. Ainsi, les matériaux issus de la photosynthèse, à savoir les plantes d'une manière générale, ont nécessairement un contenu maximum en isotope ¹⁴C.

La détermination de la teneur en biomatériau ou en bio-carbone est donnée conformément aux normes ASTM D 6866-12, la méthode B (ASTM D 6866-06) et ASTM D 7026 (ASTM D 7026-04). L'huile hydrocarbonée de la composition émolliente selon l'invention présente une teneur en biomatériau d'au moins 90%. Cette teneur est avantageusement plus élevée, en particulier supérieure ou égale à 95%, préférablement supérieure ou égale à 98 %et avantageusement égale à 100%.

En plus d'une teneur particulièrement élevée en biomatériau, l'huile hydrocarbonée de la composition émolliente selon l'invention possède une biodégradabilité particulièrement bonne. La biodégradation d'un produit chimique organique se réfère à la réduction de la complexité des composés chimiques grâce à l'activité métabolique de micro-organismes. Dans des conditions aérobies, les micro-organismes transforment les substances organiques en dioxyde de carbone, eau et biomasse. La méthode OCDE 306, est utilisée pour l'évaluation de la biodégradabilité des substances individuelles dans l'eau de mer. Selon cette méthode, l'huile hydrocarbonée a une biodégradabilité à 28 jours d'au moins 60%, de préférence d'au moins 70%, plus préférablement d'au moins 75% et avantageusement d'au moins 80%.

### Miscibilité de la composition émolliente :

Outre ses propriétés physico-chimiques et sensorielles améliorées dues à la composition intrinsèque de l'huile hydrocarbonée, la composition émolliente selon l'invention présente une très bonne miscibilité avec les autres corps gras classiquement utilisés dans les domaines cosmétique, dermatologique ou encore pharmaceutique.

En particulier, la composition émolliente selon l'invention présente une bonne miscibilité avec les corps gras choisis dans le groupe comprenant : les huiles hydrocarbonées d'origine biologique ou pétrochimique, les huiles végétales, les beurres végétaux, les éthers et alcools gras, les esters huileux et alcanes et les huiles silicones.

Les huiles hydrocarbonées sont des corps gras issus de procédés pétrochimiques. A titre d'exemple, on peut citer, les huiles minérales, les isoparaffines, les cires, les paraffines, les polyisobutènes ou encore les polydécènes.

Des exemples d'huiles végétales sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ou de camélia.

Les beurres végétaux sont des corps gras qui ont les mêmes propriétés que les huiles végétales. La différence entre les deux consiste dans le fait que les beurres se présentent sous forme solide à température ambiante. Aussi, contrairement aux huiles végétales, la matière première dont est extrait un beurre (pulpe, graines ou amandes) est chauffée après avoir été broyée pour l'extraction de la matière grasse. Comme les huiles végétales, les beurres peuvent être raffinés pour assurer une meilleure conservation, neutraliser les odeurs, améliorer la couleur et la consistance. Riches en antioxydants et nourrissants les propriétés cosmétiques des beurres végétaux améliorent l'élasticité de la peau, protègent des agressions extérieures en laissant un film protecteur sur l'épiderme et réduisant ainsi la déshydratation, réparent et apaisent en régénérant le film hydrolipidique naturel de la peau. Des exemples de beurres végétaux sont notamment le beurre de karité, le beurre de cacao, le beurre de mangue, le beurre de shorea ou encore le beurre d'olive.

Les éthers et les alcools gras sont des substances cireuses grasses à longue chaîne et aux propriétés remarquables notamment filmogènes, émollientes, hydratantes, adoucissantes et protectrices. Ils agissent comme huiles hydratantes et comme émulsifiants. Des exemples d'alcool gras ou d'éthers sont : le cetyl Alcohol, le Stearyl Alcohol, le myristyl alcohol, l'auryl alcohol, le behenyl alcohol, le cetearyl alcohol, le dicaprylyl éthers, les stearyl éthers ou l'octyldodecanol (identifiés par leur dénomination INCI).

Les esters huileux ou huiles estérifiées sont le produit d'une réaction entre des acides gras (acides à chaînes plus longues, comme par exemple l'acide stéarique, l'acide oléique, l'acide palmitique) et des alcools (des alcools gras ou des polyols comme le glycérol). Ces huiles peuvent contenir des substances issues de la pétrochimie, comme c'est le cas pour l'Isopropyl Palmitate. Des exemples d'esters huileux sont le caprylic capric triglyceride, le coco caprylate caprate, l'oleyl erucate, l'oleyl linoleate, le decyl oleate ou encore le PPG-3 benzyl éther myristate (identifiés par leur dénomination INCI).

On entend par huiles de silicones ou polysiloxanes, une huile comprenant au moins un atome de silicium, et notamment au moins un groupe Si-O. Comme huile de silicone, on peut notamment citer le phenylpropyldimethylsiloxysilicate, les dimethicones ou encore le cyclopentasiloxane (identifiés par leur dénomination INCI).

### Additifs :

La composition émolliente selon l'invention peut également être mise en mélange avec tout adjuvant ou additif habituellement utilisé dans les domaines considérés et notamment dans les domaines cosmétique, dermatologique ou pharmaceutique. Bien entendu, l'homme du métier veillera à choisir le ou les éventuels additifs de la composition selon l'invention de manière telle que les propriétés avantageuses attachées intrinsèquement à la composition émolliente conforme à l'invention ne soient pas ou substantiellement pas, altérées par l'addition envisagée. Parmi les adjuvants classiques susceptibles d'être contenus (selon le caractère hydrosoluble ou liposoluble de ces adjuvants), on peut citer notamment les tensioactifs moussants anioniques (tels que lauryl ether sulfate de sodium, alkyl phosphate de sodium, trideceth sulfate de sodium), amphotères (tels que alkyl bétaine, disodium cocoamphodiacetate) ou non ioniques de HLB supérieure à 10 (tels que POE/PPG/POE, Alkylpolyglucoside, polyglycery1-3hydroxylauryl ether) ; les conservateurs ; les séquestrants (EDTA) ; les antioxydants ; les parfums ; les matières colorantes telles que les colorants solubles, les pigments et les nacres ; les charges matifiantes, tenseurs, blanchissantes ou exfoliantes ; les filtres solaires ; les actifs cosmétiques ou dermatologiques et agents ayant pour effet d'améliorer les propriétés cosmétiques de la peau, hydrophiles ou lipophiles ; les électrolytes ; les polymères hydrophiles ou lipophiles, anioniques, non ioniques, cationiques ou amphotères, épaississants, gélifiants ou dispersants. Les quantités de ces différents adjuvants sont celles classiquement utilisées dans le domaine considéré, et par exemple de 0,01 à 20 % du poids total de la composition. Comme actifs utilisables dans la composition émolliente de l'invention, on peut citer par exemple, les vitamines hydrosolubles ou liposolubles comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; les antiseptiques ; les actifs antibactériens comme le 2,4,4'-trichloro-2'-hydroxy diphényl éther (ou triclosan), le 3,4,4'-trichlorocarbanilide (ou triclocarban) ; les antisébohrréïques ; les antimicrobiens tels que le peroxyde de benzoyle, la niacine (vit. PP) ; les amincissants tels que la caféine ; les azurants optiques, et tout actif approprié pour le but final de la composition, et leurs mélanges.

La présente invention a donc également pour objet une composition cosmétique, dermatologique ou pharmaceutique comprenant la composition émolliente décrite précédemment, au moins un corps gras choisi parmi : les huiles végétales, les beurres végétaux, les éthers et alcools gras, les esters huileux et alcanes et les huiles silicones et au moins un additif choisi parmi les additifs précités.

Cette composition cosmétique, dermatologique ou pharmaceutique comprend un milieu physiologiquement acceptable, c'est-à-dire qui ne présente pas d'effets secondaires délétères et en particulier qui ne produit pas de rougeurs, d'échauffements, de tiraillements ou de picotements inacceptables pour un utilisateur. Ce milieu comprend éventuellement de l'eau et/ou au moins une huile en tant que corps gras, en plus de la composition émolliente précitée.

Selon un mode de réalisation la composition cosmétique, dermatologique ou pharmaceutique a une teneur en composition émolliente telle que décrite allant de 0,5 à 80%, de préférence de 1 à 50% et avantageusement de 5 à 30% en poids par rapport au poids total de la composition.

La composition cosmétique, dermatologique ou pharmaceutique selon l'invention peut ainsi être une composition anhydre, une émulsion telle qu'une émulsion eau-dans-huile (E/H), une émulsion huile-dans-eau (H/E) ou une émulsion multiple (notamment E/H/E ou H/E/H), une nano-émulsion, ou encore une dispersion.

La composition cosmétique, dermatologique ou pharmaceutique selon l'invention se présente sous forme de crème plus ou moins souple ou d'une émulsion vaporisable, elle peut constituer par exemple une composition de démaquillage ou de nettoyage de la peau, des lèvres, une composition solaire (protection U.V.), ou après-solaire, une composition pour le massage de la peau, une composition de baume soin douche, une composition anti-transpirante, une composition de masque, une composition de baume réparateur, une composition gommante et/ou exfoliante aussi bien pour le visage que pour les mains (lorsqu'elle contient des particules exfoliantes), une composition de maquillage, une composition de rasage, une composition de baume après-rasage, une composition parfumée, une composition pour lingettes ou encore une composition vaporisable.

La composition de l'invention se caractérise avantageusement par le fait qu'elle présente une stabilité d'une durée supérieure ou égale à 4 semaines, avantageusement supérieure ou égale à 6 semaines, la stabilité étant évaluée après un stockage sans agitation à température ambiante, à 40°C et à 50°C et correspondant à une évaluation visuelle de la coloration et de l'aspect ainsi qu'une évaluation olfactive et/ou une mesure de la viscosité.

### Utilisation de la composition émolliente :

L'invention a encore pour objet l'utilisation cosmétique, dermatologique ou pharmaceutique de la composition telle que définie ci-dessus pour une application topique.

L'invention a aussi pour objet l'utilisation cosmétique, dermatologique ou pharmaceutique de la composition telle que définie ci-dessus comme produit de soin de la peau (sérums, crèmes, baumes, etc) comme produit d'hygiène, comme produit solaire/après-solaire, comme produit de maquillage, comme produit démaquillant, comme produit parfumé, comme produit anti-transpirant.

L'invention a encore pour objet un procédé cosmétique, dermatologique ou pharmaceutique de traitement de la peau, comprenant au moins une étape d'application sur la peau d'une composition telle que définie ci-dessus.

La composition de l'invention peut également être utilisée pour la formulation de compositions cosmétiques, de compositions dermatologiques ou de compositions pharmaceutiques comprenant d'autres composants ou d'autres phases que ceux décrits ci-dessus. Il peut s'agir notamment de la formulation de compositions de soin, d'hygiène, de maquillage.

### Procédé de traitement cosmétique :

Enfin, l'invention couvre aussi un procédé de traitement cosmétique comprenant au moisn une étape d'application, de préférence par étalement, sur la peau des compositions selon l'invention.

### EXEMPLES

Dans la suite de la présente description, des exemples sont donnés à titre illustratif de la présente invention et ne visent en aucun cas à en limiter la portée.

Différentes compositions émollientes biosourcées selon l'invention et une composition émolliente classiquement retrouvée dans le domaine de l'invention ont été évaluées pures ou en formulation. Cette évaluation porte à la fois sur le potentiel sensoriel de ces compositions émollientes pures ou en formulation ainsi que sur leur stabilité au cours du temps.

Les différentes formulations évaluées sont une émulsion huile dans l'eau et une huile sèche comprenant au moins une composition émolliente selon l'invention.

### Compositions émollientes:

Le tableau 1 regroupe les propriétés physico chimiques des différentes compositions émollientes évaluées.

Les compositions A, B et C sont des compositions émollientes biosourcées selon l'invention. La composition D est une composition émolliente comparative commercialisée par la société Croda sous le nom commercial Arlamol HD. Cette composition émolliente est un isohexadécane issue de la pétrochimie et classiquement retrouvé dans le marché de la cosmétique par exemple.

**Tableau1**

| Caractéristiques | Composition émolliente A (selon l'invention) | Composition émolliente B (selon l'invention) | Composition émolliente C (selon l'invention) | Composition émolliente D (Exemple comparatif - Arlamol HD) |
|---|---|---|---|---|
| Aromatiques (ppm) | <20 | <20 | <20 | <1 |
| Soufre (ppm) | 0.1 | 0.1 | 0.11 | |
| % iso paraffines (w/w) | 98.9 | 95.1 | 96.2 | >99 |
| % n-paraffines (w/w) | 1.1 | 4.9 | 3.8 | <1 |
| % naphténiques (w/w) | 0 | 0 | 0 | 0 |
| C13 (iso) | 0.66 | 0 | 0 | 0 |
| C14 (iso) | 4.15 | 0.12 | 0 | 0 |
| C15 (iso) | 48.35 | 11.45 | 0 | 0 |
| C16 (iso) | 42.80 | 47.89 | 1.58 | 100 |
| C17 (iso) | 2.52 | 18.57 | 14.17 | 0 |
| C18 (iso) | 0.38 | 17.07 | 79.69 | 0 |
| C19 (iso) | 0 | 0 | 0.12 | 0 |
| C20 (iso) | 0 | 0 | 0.38 | 0 |
| C27 (iso) | 0 | 0 | 0.29 | 0 |
| Quantité de carbones d'origine biologique (%) | 97 | 97 | 98 | 0 |
| Point d'ébullition initial (°C) | 247.0 | 259.5 | 293.6 | 210 |
| Point ébullition 5% (°C) | 255.7 | 270.2 | 296.7 | 230 |
| Point ébullition 50% (°C) | 258.9 | 274.5 | 298.5 | |
| Point ébullition 95% (°C) | 266.8 | 286.4 | 305.3 | 238 |
| Point d'ébullition final (°C) | 269.0 | 287.5 | 324.1 | 250 |
| Biodégradabilité OECD (28 jours) (%) | 80 | 83 | 83 | <60 |
| Indice de réfraction à 20°C | 1,4336 | 1,4357 | 1,4394 | 1,439 |
| densité à 15°C (kg/m3) | 776,4 | 780,3 | 787,2 | 790 |
| Point éclair (°C) | 115 | 125 | 149 | 102 |
| Viscosité Cinématique à 40°C (cSt) | 2,49 | 2,94 | 3,87 | 4,2 |
| Pression de vapeur à 20°C (kPa) | <0,01 | <0,01 | <0,01 | 0,01 |

### Evaluation des compositions émollientes pures :

Les compositions émollientes pures sont évaluées en analyse sensorielle et organoleptique. Les critères d'évaluation sont les suivants :
- Odeur intrinsèque en masse : odeur dégagée par la composition lorsque le flacon est ouvert (pas d'application effectuée).
- Diffusion sur la peau: une goutte est laissée sur la peau pendant 30 secondes. Au bout de 30 secondes, la capacité de la goutte à s'être diffusée ou étalée sans aide manuelle est évaluée.
- Etalement : la composition présente-t-elle une résistance au mouvement lorsqu'elle est appliquée.
- Brillance sur la peau : un film brillant persiste-t-il sur la peau une fois la composition appliquée.
- Crissant entre les doigts : une fois la composition appliquée, un résidu persistant est-il perçu entre les doigts et s'ils sont frottés ensemble, un bruit est-il audible.
- Gras: (critère évalué sur la peau 1 minute après l'étalement) lorsque la peau est placée entre le pouce et l'index, et qu'un mouvement de frottement est effectué, y a-t-il de la résistance. La composition facilite-t-elle le déplacement des deux doigts tout en donnant un aspect huileux à la peau.
- Pénétration: (critère évalué sur la peau 2 minutes après l'étalement) en effectuant un glissement sur la peau, la composition a-t-elle disparu et aucun résidu n'est récupéré.
- Odeur rémanente sur la peau: (critère évalué sur la peau 2 minutes après l'étalement) Après 2 minutes d'application, une odeur persistante est-elle perçue sur la peau.

L'analyse sensorielle et organoleptique est effectuée par un panel de 5 personnes. L'analyse est effectuée sur le dessous de l'avant-bras en déposant une à deux gouttes de chaque composition émolliente à l'aide d'une pipette en verre, la composition est ensuite appliquée à l'aide des doigts. Le nombre de gouttes appliquées est reproductible pour un même testeur. 4 essais sont effectués pour chacune des compositions. Un système de cotation (de 0 à 5 du moins prononcé au plus prononcé) est mis en place pour faciliter l'exploitation.

Le tableau 2 reprend les moyennes des résultats de cotation obtenues pour chaque critère sensoriel évalué pour les différentes compositions émollientes ; dans ce tableau sur la caractéristique de l'odeur, c'est l'odeur rémanente qui est la caractéristique la plus importante.

**Tableau 2**

| | Composition émolliente A | Composition émolliente B | Composition émolliente C | Composition émolliente D |
|---|---|---|---|---|
| odeur intrinsèque en masse | 3,5 | 2,8 | 4,2 | 0,7 |
| diffusion sur la peau | 2,8 | 2,9 | 1,8 | 3,7 |
| étalement | 4 | 3,7 | 3,1 | 3,8 |
| brillance sur la peau | 3,1 | 1,7 | 2,2 | 2,5 |
| crissant entre les doigts | 1,4 | 1,6 | 1 | 1,7 |
| gras | 3,1 | 2,3 | 3 | 3 |
| pénétration | 2,1 | 3,4 | 2,2 | 2,8 |
| odeur rémanente | 1,7 | 0,9 | 1,2 | 0,5 |

On constate que les compositions émollientes A, B et C ont un profil sensoriel sensiblement comparable à celui de la composition émolliente D comparative.

### Compositions émollientes en formulation :

### Emulsion huile-dans-l'eau (H/E)

Des émulsions huile dans l'eau (H/E) classiques et non parfumées sont formulées avec chacune des compositions émollientes exemplifiées.

Les émulsions H/E exemplifiées comprennent les différentes compositions émollientes A, B, C et D dans des proportions de 0%, 5%, 10% et 15% en poids par rapport au poids total de l'émulsion. La quantité totale d'émollient restant constante à 15% dans les formulations, les compositions émollientes A, B et C sont mises en mélange avec la composition émolliente comparative D.

Trois émulsions de 500g sont ainsi formulées avec chacune des compositions A, B et C. 10 émulsions H/E sont ainsi évaluées au total (l'émulsion H/E numéro 10 comprenant la composition émolliente comparative D à une teneur de 15% étant l'émulsion comparative pour chacune des formulations).

La formulation des émulsions H/E est réalisée à l'aide d'un Supertest et d'un turrax qui permettent d'homogénéiser l'émulsion en affinant la taille des gouttelettes.

La structure de l'émulsion H/E évaluée reste la même pour tous les essais. Seules les quantités des différentes compositions émollientes de la phase grasse varient.

Le tableau 3 ci-dessous indique les formulations des émulsions H/E selon les exemples. Les pourcentages indiqués correspondent au poids en matière active par rapport au poids total de l'émulsion. Pour toutes les émulsions, la base de la phase grasse est la même et la phase aqueuse est identique dans tous les cas.

**Tableau 3**

| Phases | | Nom commercial | Emulsion 1 | Emulsion 2 | Emulsion 3 | Emulsion 4 | Emulsion 5 | Emulsion 6 | Emulsion 7 | Emulsion 8 | Emulsion 9 | Emulsion 10 |
|---|---|---|---|---|---|---|---|---|---|---|---|---|
| PHASE GRASSE | Phase A | Simulsol 165 (émulsionnant) | 3,50% | 3,50% | 3,50% | 3,50% | 3,50% | 3,50% | 3,50% | 3,50% | 3,50% | 3,50% |
| | | Montanov 68 (émulsionnant) | 1,50% | 1,50% | 1,50% | 1,50% | 1,50% | 1,50 % | 1,50% | 1,50% | 1,50% | 1,50% |
| | | Lanette O OR (régulateur de viscosité) | 0,50% | 0,50% | 0,50% | 0,50% | 0,50% | 0,50 % | 0,50% | 0,50% | 0,50% | 0,50% |
| | | Karité CP (corps gras) | 2,00% | 2,00% | 2,00% | 2,00% | 2,00% | 2,00 % | 2,00% | 2,00% | 2,00% | 2,00% |
| | | Dub DSPE (agent viscosant) | 0,60% | 0,60% | 0,60% | 0,60% | 0,60% | 0,60 % | 0,60% | 0,60% | 0,60% | 0,60% |
| | | Composition émolliente A | 5,00% | 10,00% | 15,00% | - | - | - | - | - | - | 0,00% |
| | | Composition émolliente B | - | - | - | 5,00% | 10,00% | 15,00% | - | - | - | - |
| | | Composition émolliente C | - | - | - | - | - | - | 5,00% | 10,00% | 15,00% | - |
| | | Composition émolliente D | 10,00% | 5,00% | 0,00% | 10,00% | 5,00% | 0,00 % | 10,00% | 5,00% | 0,00% | 15,00% |
| PHASE AQUEUSE | Phase B | Eau Déminéralisée | 69,25% | 69,25% | 69,25% | 69,25% | 69,25% | 69,25% | 69,25% | 69,25% | 69,25% | 69,25% |
| | | Gomme xanthane | 0,20% | 0,20% | 0,20% | 0,20% | 0,20% | 0,20 % | 0,20% | 0,20% | 0,20% | 0,20% |
| | | Glycérine | 3,00% | 3,00% | 3,00% | 3,00% | 3,00% | 3,00% | 3,00% | 3,00% | 3,00% | 3,00% |
| | | Carbopol Ultrez 21 (polymère) | 0,20% | 0,20% | 0,20% | 0,20% | 0,20% | 0,20% | 0,20% | 0,20% | 0,20% | 0,20% |
| | Phase C | Phenoxethol (conservateur) | 0,80% | 0,80% | 0,80% | 0,80% | 0,80% | 0,80% | 0,80% | 0,80% | 0,80% | 0,80% |
| | | Solution aqueuse de NaOH à 10% | 0,30% | 0,30% | 0,30% | 0,30% | 0,30% | 0,30% | 0,30% | 0,30% | 0,30% | 0,30% |
| | Phase D | Eau Déminéralisée | 2,00% | 2,00% | 2,00% | 2,00% | 2,00% | 2,00% | 2,00% | 2,00% | 2,00% | 2,00% |
| | | Hydrolite-5 (stabilisateur) | 1,00% | 1,00% | 1,00% | 1,00% | 1,00% | 1,00 % | 1,00% | 1,00% | 1,00% | 1,00% |
| | | Chlorphenesin BP 73 (conservateur) | 0,25% | 0,25% | 0,25% | 0,25% | 0,25% | 0,25 % | 0,25% | 0,25% | 0,25% | 0,25% |

### Mode de préparation :

- L'eau de la phase B est pesée. Sans agitation, le Carbopol Ultrez 21 est saupoudré dans l'eau pour hydratation pendant 10 minutes sans agitation. Ensuite, le mélange est mis sous agitation et chauffée à 50°C. A 50°C, un prémix est réalisé entre la Rhodicare T et la glycérine puis il est versé dans l'eau sous vive agitation. L'agitation est maintenue jusqu'à l'obtention d'une phase homogène. Enfin la chauffe est poursuivie à 75°C.
- La phase A est pesée, mise sous agitation et chauffée à 75°C.
- A 75°C, sous vive agitation (1150 rpm), la phase A est versée progressivement dans la phase B. Le refroidissement est alors amorcé.
- A 72°C l'émulsion est passée au turrax 30 secondes à 9500rpm.
- Le refroidissement est alors poursuivi jusqu'à 50°C sous agitation modérée (500 rpm). A 50°C, sous agitation, les composants de la phase C sont incorporés un à un dans l'émulsion. Le refroidissement est poursuivi jusqu'à 30°C.
- A 30°C, sous agitation, la phase D (préalablement agitée jusqu'à l'obtention d'une phase homogène) est versée dans l'émulsion. Le pH est ajusté si nécessaire (avec une solution de soude à 10%) entre 5,50 et 6,00.

### Evaluation des émulsions huiles dans l'eau (H/E) :

### Stabilité :

L'étude de stabilité des émulsions H/E est réalisée à température ambiante (TA) du jour, à 40°C et à 50°C pendant un mois par évaluation visuelle et olfactive de l'aspect, de la couleur et de l'odeur ainsi que par évaluation de la viscosité des émulsions au cours du temps.

Aucune modification d'aspect ou de couleur ni aucune apparition d'odeur n'ont été constatées lors de l'évaluation visuelle et olfactive des 10 émulsions huiles dans l'eau formulées.

Les viscosités sont mesurées à l'aide de l'appareillage de Brookfield RV DVII+PRO, mobile 6 à 20 tours par minute, pendant 1 minute dans des pots de 200g ayant été préalablement thermostatés entre 24,5 et 25,5°C.

Le tableau 4 indique les résultats de l'évaluation de la viscosité des 10 émulsions H/E.

**Tableau 4**

| | | Emulsion 1 | Emulsion 2 | Emulsion 3 | Emulsion 4 | Emulsion 5 | Emulsion 6 | Emulsion 7 | Emulsion 8 | Emulsion 9 | Emulsion 10 |
|---|---|---|---|---|---|---|---|---|---|---|---|
| Viscosité (mPA.s) | JO | 34150 | 32900 | 35100 | 30750 | 24150 | 30350 | 29350 | 32400 | 28600 | 30600 |
| | J+1 | 32250 | 33500 | 34300 | 35100 | 30300 | 34300 | 32050 | 36450 | 31750 | 30900 |
| | 2 semaines | 34550 | 36150 | 41050 | 35300 | 30450 | 34950 | 32400 | 37150 | 33350 | 34950 |
| | 4 semaines | 34300 | 36900 | 42100 | 37950 | 31200 | 34900 | 33450 | 34900 | 31150 | 35550 |
| | 8 semaines | 37500 | 38000 | 43200 | 35700 | 31650 | 34250 | 34050 | 37450 | 35100 | 35950 |

On observe une viscosité similaire pour toutes les émulsions H/E formulées. La viscosité des émulsions reste stable au cours du temps. (Une très légère augmentation générale de la viscosité au bout de 8 semaines est constatée pour l'ensemble des 10 émulsions H/E. Elle est considérée comme non significative car liée au mûrissement des émulsions).

### Analyse sensorielle :

Les 10 émulsions H/E formulées sont également évaluées en analyse sensorielle. Les critères d'évaluation sont alors adaptés à la galénique et sont les suivants :
- Odeur intrinsèque en masse : odeur dégagée par l'émulsion lorsque l'on ouvre le flacon (pas d'application effectuée).
- Etalement : l'émulsion présente-t-elle une résistance au mouvement lorsqu'elle est appliquée.
- Brillance sur la peau : un film brillant persiste-t-il sur la peau une fois l'émulsion appliquée.
- Vitesse de pénétration : Il s'agit d'évaluer si la pénétration de l'émulsion est plus rapide ou plus lente selon l'échantillon. En effectuant un glissement sur la peau, l'émulsion -t-elle disparu et aucun résidu n'est récupéré.
- Gras (après pénétration): y -t-il une résistance lorsque la peau est placée entre le pouce et l'index et qu'un mouvement de frottement est effectué. L'émulsion facilite-t-elle le déplacement des deux doigts tout en donnant un aspect huileux à la peau.
- Douceur (après pénétration): Quelle est la douceur ressentie lorsque la paume de la main est passée sur la zone d'étalement de l'émulsion. La peau est-elle plus douce sur la zone d'étalement.
- Collant (après énétration): une adhérence de la peau est-elle ressentie lorsque la paume de la main est posée sur la zone d'étalement de l'émulsion, puis enlevée. La peau est-elle plus collante.
- Odeur rémanente sur la peau (2min) : (critère évalué sur la peau 2 minutes après l'étalement) Après 2 minutes d'application, une odeur persistante est-elle perçue sur la peau.

Comme pour l'analyse sensorielle des compositions émollientes pures, un système de cotation (de 0 à 5 du moins prononcé au plus prononcé) est mis en place pour faciliter l'exploitation.

Le tableau 5 reprend les résultats des moyennes de cotation de chacun des critères sensoriels évalués pour les différentes émulsions H/E.

**Tableau 5**

| | Emulsion 1 | Emulsion 2 | Emulsion 3 | Emulsion 4 | Emulsion 5 | Emulsion 6 | Emulsion 7 | Emulsion 8 | Emulsion 9 | Emulsion 10 |
|---|---|---|---|---|---|---|---|---|---|---|
| odeur intrinsèque en masse | 1,7 | 1,4 | 1,6 | 1,2 | 1,2 | 1,2 | 1,7 | 1,8 | 1,7 | 1,8 |
| étalement | 2,7 | 3,7 | 3,1 | 2,7 | 2,6 | 2,9 | 2,7 | 3,1 | 3,2 | 2,2 |
| brillance sur la peau | 1,5 | 1,6 | 1,5 | 1,7 | 1,4 | 1,7 | 1,3 | 1,6 | 1,6 | 1,2 |
| vitesse de pénétration | 2,8 | 2,2 | 2,3 | 2,6 | 2,7 | 2,4 | 2,4 | 2,6 | 2,3 | 2,7 |
| gras | 1,8 | 2,1 | 1,9 | 1,7 | 1,2 | 1,5 | 2,1 | 1,7 | 2 | 1.4 |
| douceur | 2,2 | 2 | 2,5 | 2 | 2,7 | 2,5 | 2,5 | 2,5 | 2,2 | 2 |
| collant | 0,8 | 0,9 | 1 | 0,3 | 0,8 | 1 | 1,1 | 0,9 | 1,4 | 1 |
| odeur rémanente | 1,7 | 1,6 | 1,75 | 1,3 | 1,4 | 1,5 | 1,6 | 1,5 | 1,6 | 1,8 |

On constate que l'ensemble des émulsions comprenant les compositions émollientes A, B et C a une odeur rémanente et une odeur intrinsèque moins importante, un meilleur étalement, une douceur plus importante et une brillance sur la peau plus prononcée que l'émulsion 10 qui comprend la composition émolliente comparative D. Ces résultats avec des compositions formulées complètes présentent une amélioration remarquable par rapport à ceux obtenus pour les compositions émollientes pures.

### Galénique anhydride (huiles sèches)

Des huiles sèches pour le corps, non parfumées, contenant 30% de composition émolliente au total sont formulées avec chacune des compositions émollientes exemplifiées.

Les huiles pour le corps formulées comprennent les différentes compositions émollientes A, B et C dans des proportions de 10%, 20% et 30%. La quantité totale de composition émolliente restant constante dans les formulations, les compositions émollientes A, B et C sont mises en mélange avec la composition comparative D.

Trois huiles sèches de 300g sont formulées avec chacune des compositions émollientes A, B et C.

10 huiles sèches pour le corps ont donc été évaluées au total (l'huile sèche numéro 10 comprenant la composition émolliente comparative D à une teneur de 30% étant l'huile comparative pour chacune des formulations).

Le tableau 6 ci-dessous indique les formulations des huiles sèches selon les exemples. Les pourcentages indiqués correspondent au poids en matière active par rapport au poids total de la composition.

**Le tableau 6**

| | Huile sèche 1 | Huile sèche 2 | Huile sèche 3 | Huile sèche 4 | Huile sèche 5 | Huile sèche 6 | Huile sèche 7 | Huile sèche 8 | Huile sèche 9 | Huile sèche 10 |
|---|---|---|---|---|---|---|---|---|---|---|
| Dub MCT 55/45 ester constituant l'ossature de la formulation anhydride | 35,00 % | 35,00 % | 35,00 % | 35,00 % | 35,00 % | 35,00 % | 35,00 % | 35,00 % | 35,00 % | 35,00 % |
| Dub IPP ester constituant l'ossature de la formulation anhydride | 35,00 % | 35,00 % | 35,00 % | 35,00 % | 35,00 % | 35,00 % | 35,00 % | 35,00 % | 35,00 % | 35,00 % |
| Composition émolliente A | 10,00 % | 20,00 % | 30,00 % | - | - | - | - | - | - | - |
| Composition émolliente B | 0,00% | 0,00% | 0,00% | 10,00 % | 20,00 % | 30,00 % | - | - | - | - |
| Composition émolliente C | - | - | - | - | - | - | 10,00 % | 20,00 % | 30,00 % | - |
| Composition émolliente D | 20,00 % | 10,00 % | 0,00% | 20,00 % | 10,00 % | 0,00% | 20,00 % | 10,00 % | 0,00% | 30,00 % |

Mode de préparation : Les composants sont pesés puis mis sous agitation jusqu'à l'obtention d'une phase homogène.

### Evaluation des huiles sèches pour le corps

### Stabilité :

L'étude de stabilité des 10 huiles pour le corps formulées est réalisée à température ambiante (TA) du jour, à 40°C et à 50°C pendant un mois par évaluation visuelle et olfactive de l'aspect, de la couleur et de l'odeur.

Comme pour l'analyse de la stabilité des 10 émulsions huile-dans-l'eau, aucune modification d'aspect ou de couleur ni aucune apparition d'odeur n'ont été constatées lors de l'évaluation visuelle et olfactive des 10 huiles sèches formulées.

### Analyse sensorielle :

Les 10 huiles sèches pour le corps obtenues sont également évaluées en analyse sensorielle. Les critères d'évaluation sont alors adaptés à la galénique et sont les suivants :
- Odeur intrinsèque en masse : odeur dégagée par l'huile sèche lorsque le flacon est ouvert (pas d'application effectuée)
- Diffusion sur la peau: une goutte est laissée sur la peau pendant 30 secondes. Au bout de 30 secondes, la capacité de la goutte à s'être diffusée ou étalée sans aide manuelle est évaluée.
- Brillance sur la peau : une fois l'huile sèche appliquée, un film brillant persiste-il sur la peau.
- Gras: (critère évalué sur la peau 1 minute après l'étalement) lorsque la peau est placée entre le pouce et l'index, et qu'un mouvement de frottement est effectué, y a-t-il de la résistance. L'huile sèche facilite-t-elle le déplacement des deux doigts tout en donnant un aspect huileux à la peau.
- Pénétration: (critère évalué sur la peau 2 minutes après l'étalement) en effectuant un glissement sur la peau, l'huile sèche a-t'elle disparue et aucun résidu n'est récupéré.
- Odeur rémanente sur la peau: (critère évalué sur la peau 2 minutes après l'étalement) Après 2 minutes d'application, une odeur persistante est-elle perçue sur la peau.

Comme pour l'analyse sensorielle des compositions émollientes pures et de la formulation en émulsion H/E, un système de cotation (de 0 à 5 du moins prononcé au plus prononcé) est mis en place pour faciliter l'exploitation.

Le tableau 7 reprend les résultats des moyennes de cotation de chacun des critères sensoriels évalués pour les différentes huiles sèches pour le corps.

**Tableau 7**

| | Huile sèche 1 | Huile sèche 2 | Huile sèche 3 | Huile sèche 4 | Huile sèche 5 | Huile sèche 6 | Huile sèche 7 | Huile sèche 8 | Huile sèche 9 | Huile sèche 10 |
|---|---|---|---|---|---|---|---|---|---|---|
| odeur intrinsèque en masse | 0,6 | 1,2 | 0,9 | 0,5 | 0,8 | 1,2 | 1,5 | 2 | 1,8 | 1,6 |
| diffusion sur la peau | 2,3 | 2,2 | 2,1 | 2,3 | 2,1 | 1,9 | 2,6 | 2,2 | 2,2 | 2,5 |
| brillance sur la peau | 2,6 | 2,1 | 2,2 | 2,2 | 2,5 | 2,3 | 2 | 1,9 | 2,4 | 1,8 |
| gras | 2,4 | 2 | 2,5 | 2,7 | 3 | 2,5 | 2,9 | 2,4 | 3,1 | 2,3 |
| pénétration | 2,7 | 3 | 2,9 | 2,8 | 2,7 | 2,8 | 2,4 | 2,4 | 2,4 | 3,1 |
| odeur rémanente | 0,5 | 0,3 | 0,4 | 0,3 | 0,5 | 0,7 | 0,8 | 0,9 | 1,3 | 1 |

On constate que toutes les huiles sèches comprenant les compositions émollientes A, B et C ont une brillance sur la peau plus prononcée que l'huile sèche 10 qui comprend la composition émolliente comparative D. Les huiles sèches 1 à 8 ont également une odeur rémanente et une odeur intrinsèque moins importante que l'huile sèche comparative 10.

De manière générale, on constate que les compositions émollientes A, B et C ont des profils sensoriels très proches que ce soit en galénique anhydre type huile sèche ou en émulsion type émulsions H/E. Ces profils sensoriels sont par ailleurs supérieurs aux profils sensoriels des produits comparatifs commerciaux.

## Revendications

1. Composition émolliente comprenant au moins une huile hydrocarbonée qui comprend une teneur en poids d'isoparaffines allant de 90 à 100%, une teneur en poids de paraffines normales allant de 0 à 10% et une teneur en carbone d'origine biologique supérieure ou égale à 90 % par rapport au poids total de l'huile hydrocarbonée.

2. Composition émolliente selon la revendication 1 dans laquelle l'huile hydrocarbonée comprend une teneur en carbone d'origine biologique supérieure ou égale à 95%, de préférence supérieure ou égale à 98% et préférentiellement de 100%.

3. Composition émolliente selon l'une quelconque des revendications précédentes dans laquelle l'huile hydrocarbonée comprend une teneur en poids d'isoparaffines allant de 90 à 100%, de préférence de 95 à 100% et préférentiellement de 98% à 100% par rapport au poids total de l'huile hydrocarbonée.

4. Composition émolliente selon l'une quelconque des revendications précédentes dans laquelle l'huile hydrocarbonée est choisie parmi les isoparaffines non-cycliques comprenant de 14 à 18 atomes de carbone.

5. Composition émolliente selon l'une quelconque des revendications dans laquelle l'huile hydrocarbonée comprend une teneur en poids de paraffines normales inférieure ou égale à 10, de préférence inférieure ou égale à 5% et préférentiellement inférieure ou égale à 2% par rapport au poids total de l'huile hydrocarbonée.

6. Composition émolliente selon l'une quelconque des revendications précédentes dans laquelle l'huile hydrocarbonée comprend une teneur en poids de composés naphténiques inférieure ou égale à 1%, de préférence inférieure ou égale à 0,5% et préférentiellement inférieure ou égale à 100ppm par rapport au poids total de l'huile hydrocarbonée.

7. Composition émolliente selon l'une quelconque des revendications précédentes dans laquelle l'huile hydrocarbonée comprend une teneur en poids de composés aromatiques inférieure ou égale à 500 ppm, de préférence inférieure ou égale à 300 ppm et préférentiellement inférieure ou égale à 100 ppm par rapport au poids total de l'huile hydrocarbonée.

8. Composition émolliente selon l'une quelconque des revendications précédentes dans laquelle l'huile hydrocarbonée a une température d'ébullition allant de 230 à 340°C, de préférence de 235 à 330°C et plus préférentiellement de 240 à 325°C selon la norme ASTM D86.

9. Composition émolliente selon l'une quelconque des revendications précédentes dans laquelle l'huile hydrocarbonée est obtenue par un procédé d'hydrogénation catalytique à une température de 80 à 180°c et à une pression de 50 à 160 bars d'une charge d'origine biologique désoxygénée et/ou isomérisée.

10. Composition émolliente selon l'une quelconque des revendications précédentes dans laquelle l'huile hydrocarbonée a une biodégradabilité à 28 jours d'au moins 60%, de préférence d'au moins 70%, préférentiellement d'au moins 75% et encore plus préférentiellement d'au moins 80% mesurée selon la norme OECD 306.

11. Composition émolliente selon l'une quelconque des revendications précédentes dans laquelle l'huile hydrocarbonée a un point éclair supérieur ou égal à 110°C selon la norme ASTM D93.

12. Composition émolliente selon l'une quelconque des revendications précédentes dans laquelle l'huile hydrocarbonée a une pression de vapeur à 20°C inférieure ou égale à 0,01kPa.

13. Composition cosmétique, dermatologique ou pharmaceutique comprenant au moins une composition émolliente selon l'une des revendications 1 à 12, de préférence en une quantité allant de 0,5 à 80%, préférentiellement de 1 à 50% et avantageusement de 5 à 30% en poids par rapport au poids total de la composition.

14. Composition cosmétique, dermatologique ou pharmaceutique selon la revendication 13, comprenant au moins un corps gras choisi parmi : les huiles végétales, les huiles hydrocarbonées, les beurres végétaux, les éthers et alcools gras, les esters huileux et alcanes et les huiles silicones et/ou au moins un additif.

15. Utilisation cosmétique de la composition émolliente selon l'une quelconque des revendications 1 à 12 ou de la composition selon la revendication 13 ou 14 pour une application topique, notamment comme produit de soin pour la peau, comme produit de maquillage, comme produit capillaire, comme produit démaquillant, comme produit parfumé, comme produit solaire.

16. Composition émolliente selon l'une quelconque des revendications 1 à 12 ou la composition selon de la revendication 13 ou 14 pour une utilisation pharmaceutique.

17. Utilisation de la composition émolliente selon l'une quelconque des revendications 1 à 12 ou de la composition selon la revendication 13 ou 14 pour la formulation de produits cosmétiques, de produits dermatologiques ou de produits pharmaceutiques.

18. Procédé de traitement cosmétique de la peau comprenant au moins une étape d'application, de préférence par étalement, de la composition émolliente selon l'une quelconque des revendications 1 à 12 ou de la composition selon la revendication 13 ou 14.
